# EUROPÄISCHE PATENTSCHRIFT

(11) **EP 3 412 299 B1**
(45) Veröffentlichungstag und Bekanntmachung des Hinweises auf die Patenterteilung: **31.08.2022**
(21) Anmeldenummer: 17175227.2
(22) Anmeldetag: 09.06.2017
(51) Int. Cl.: A61K 36/30

(54) **EINTOPF-VERFAHREN BEI DER HERSTELLUNG VON SPEZIALEXTRAKTEN AUS SYMPHYTUM**
ONE POT METHOD USED FOR THE PRODUCTION OF SPECIAL EXTRACTS FROM SYMPHTUM
PROCÉDÉ DE MISE EN POT LORS DE LA FABRICATION D'EXTRAITS SPÉCIAUX DE SYMPHYTUM

(43) Veröffentlichungstag der Anmeldung: 12.12.2018
(73) Patentinhaber: SMC - Research AG, 9230 Arbon (CH)
(72) Erfinder: HÄCKER, Peter, 9230 Arbon (CH)
(74) Vertreter: IPS Irsch AG

(56) Entgegenhaltungen:
- EP-A1- 0 673 654
- EP-A1- 3 159 002
- CH. MAUZ: PHARMACEUTICA ACTA HELVETIAE, Bd. 60, 1985, Seiten 256-259, XP9133101,
- JANES DAMJAN ET AL: "Improved method for isolation of lycopsamine from roots of comfrey (Symphytum officinale)", NATURAL PRODUCT COMMUNICAT, NATURAL PRODUCT INC, US, Bd. 7, Nr. 7, 1. Juli 2012 (2012-07-01), Seiten 861-862, XP009188407, ISSN: 1934-578X
- N.N.: "Assessment report on Symphytum officinale L., radix", , 5 May 2015 (2015-05-05), pages 1-27, XP055695772, Retrieved from the Internet: URL:https://www.ema.europa.eu/en/documents /herbal-report/final-assessment-report-sym phytum-officinale-l-radix_en.pdf [retrieved on 2020-05-15]

## Beschreibung

Die Erfindung bezieht sich auf das Gebiet der Herstellung von Spezialextrakten. Sie bezieht sich insbesondere auf ein Verfahren zur Herstellung eines Spezialextrakts aus Symphytum, ein Spezialextrakt aus Symphytum sowie ein Verfahren zur Herstellung eines Pflanzenauszugs aus Symphytum, und ein Verfahren zur Pyrrolizinalkaloide-Reinigung von einem Pflanzenauszug aus Symphytum gemäss dem Oberbegriff der entsprechenden unabhängigen Patentansprüche.

Symphytum, auch Beinwell genannt, ist eine traditionelle Arzneipflanze, deren Wirkung bereits in der Antike bekannt war. Die antiinflammatorischen, analgetischen und geweberegenerierenden Eigenschaften werden sowohl dem Beinwellkraut, bestehend aus den frischen und/oder getrockneten oberirdischen Teilen, als auch der Beinwellwurzel, bestehend aus frischen und/oder getrockneten unterirdischen Teilen, zugeschrieben. In jüngster Vergangenheit hat sich der Indikationsbereich von Beinwellwurzelextrakten durch den Nachweis der perkutanen Wirksamkeit bei Sprunggelenksdistorsion bestätigt. Dabei erwies sich Beinwell in Bezug auf den Rückgang von Schmerz, Entzündung, Schwellung, Bewegungseinschränkung und globale Wirksamkeit als klinisch wirksam und signifikant überlegen. Darüber hinaus haben moderne Testverfahren und Anwendungsbeobachtungen die Wirksamkeit und Unbedenklichkeit bei unterschiedlichen Muskel- und Gelenkbeschwerden bestätigt. Somit hat sich die medizinische Verwendung von Präparaten aus allen, insbesondere den unterirdischen, Teilen der Pflanze (Symphytum officinale) gut etabliert und die Präparate werden neben der Behandlung von Prellungen, Zerrungen oder Verstauchungen auch bei schmerzhafter Gelenkarthrose und akuten Rückenschmerzen angewendet.

Die Bestandteile der Beinwellpflanze enthalten 0.6-4.7% Allantoin, reichlich Schleimpolysaccharide (25-30%) aufweisend Fructose- und Glucose-Einheiten, Phenolsäuren wie Rosmarinsäure (bis 0.2%), Chlorogensäure (0.012%) sowie Kaffeesäure (0.004%) und alpha-Hydroxykaffeesäure, Glycopeptide, Aminosäuren und Triterpensaponine in Form von monodesmosidischen und bidesmosidischen Glycosiden.

Als Wirkstoffe, also klinisch wirksame Bestandteile der Symphytum, kommen Allantoin, ein mit Harnsäure verwandtes Purinderivat, die Schleimpolysaccharide, Gerbstoffe und Triterpensaponine in Frage. Für die Pharmakodynamik sind Rosmarinsäure und andere Hydroxyzimtsäurenderivate von zentraler Bedeutung. Vermutlich sind alle Wirkstoffgruppen von Relevanz, die genauen Wirkmechanismen sind jedoch noch nicht vollständig geklärt.

Ebenfalls enthalten alle Pflanzenteile von Symphytum Pyrrolizidinalkaloide, kurz auch PA bzw. PAs genannt, in Form ihrer N-Oxide. Symphytum weist etwa 150 verschiedene Pyrrolizidinalkaloide (PAs) auf, die wichtigsten sind 7-Acetyllycopsamine, 7-Acetylintermedine zusammen mit geringen Mengen an Intermedine, Lycopsamin und Symphytine. Die Gesamtmenge von PAs variiert von 0.013 % bis 3 %, auch aus methodenspezifischen Gründen (Analyse). Die Pyrrolizidinalkaloide Echimidine und Symlandine werden in Symphytum officinale nicht gefunden. Bei den PAs handelt es sich möglicherweise um mutagene bzw. carcinogene Substanzen, weshalb auf die innerliche Anwendung der Droge, sowie daraus hergestellte Zubereitungen, gänzlich verzichtet wird. Die sehr beliebte äusserliche Anwendung bei intakter Haut erscheint jedoch vertretbar, weshalb in Europa derzeit behördlicherseits definierte Grenzwerte für Tagesdosen von maximal 0.35 Mikrogramm Pyrrolizidinalkaloiden toleriert werden. In jedem Fall sollte es aus toxikologischer Sicht und aus Gründen der Vorsorge wünschenswert sein, den Gehalt an PAs möglichst gering zu halten.

Im industriellen Bereich ist bisher ein Verfahren bekannt, welches darauf beruht, dass ein alkoholischer Extrakt aus Petasites hybridus zur Abreicherung der PAs mit einem Kationenaustauscher behandelt wird. Auf diese Weise konnten seiner Zeit in diesen Extrakten mehr als 93% der PAs entfernt werden (siehe Ch. Mauz et. al. in Pharmaceutica Acta Helvetiae, Band 60 (1985), 256-259). Petasites weist im Gegensatz zu Symphytum keine Schleimpolysaccharide auf.

Wie in Natural Product Communication, 2012, 7, 7, 861-862 beschrieben, kann aus einem Spezialauszug von Symphytumwurzeln Lycopsamin, ein spezifisches Pyrrolizidinalkaloid, isoliert werden. Es handelt sich im vorliegenden Fall ausschliesslich um die Gewinnung einer analytischen Vergleichssubstanz, nämlich das Lycopsamin, und nicht um die Herstellung eines Extrakts.

Das hohe durch entsprechende Studien erkannte Wirkpotential der Beinwellpflanze rechtfertigt die Optimierung einer technologisch-methodischen Extrakt-Formulierung, weshalb vorliegender Erfindung daher mindestens die Aufgabe zugrunde liegt, ein Verfahren zur Herstellung von Spezialextrakten aus Symphytum bereitzustellen, welches durch eine im Wesentlichen produktschonende Verfahrenstechnologie eine hohe Ausbeute an pharmazeutisch relevanten Inhaltsstoffen ermöglicht.

Es ist deshalb Aufgabe der Erfindung, ein vereinfachtes Verfahren zur Pyrrolizidinalkaloide-Reinigung eines Pflanzenauszugs (Primärextrakts) aus Symphytum sowie ein Verfahren zur Herstellung eines Spezialextrakts, ein Verfahren zur Herstellung eines Pflanzenauszugs (Primärextrakts) aus Symphytum und ein Spezialextrakt aus Symphytum bereitzustellen.

Diese Aufgabe löst eine ein Verfahren zur Herstellung eines Pflanzenauszugs (Primärextrakts) aus Symphytum und ein Verfahren zur Pyrrolizidinalkaloide-Reinigung in einem Dispersen Extrakt System (DES) des Primärextrakts aus Symphytum in einem Eintopf-Verfahren sowie ein Verfahren zur Herstellung eines Symphytum-Spezialextrakts aus einem Pyrrolizidinalkaloide-gereinigten Primärextrakt, sowie ein Spezialextrakt aus Symphytum mit den Merkmalen der entsprechenden unabhängigen Patentansprüche.

Das Verfahren zur Pyrrolizidinalkaloide-Reinigung von einem Pflanzenauszug (Primärextrakt) aus Symphytum weist die folgenden Schritte auf:
- Bereitstellen von einem Pflanzenauszug (Primärextrakt) aus Symphytum, wobei ein Raffinat von dem Pflanzenauszug abgetrennt wird, und wobei das Raffinat hochmolekulare Polysaccharide in einem ersten Niederschlag aufweist, und die hochmolekularen Polysaccharide aus einem Suspensions System aus Symphytum in einer ersten differentiellen Fällung ausgefällt werden, indem der pH-Wert des Suspensions Systems auf 4 - 5 eingestellt wird;
- differentielles Fällen von niedermolekularen Polysacchariden als zweiten Niederschlag aus einem nach dem Trennvorgang vom Raffinat entstandenen Pflanzenauszug (Primärextrakt), und beim differentiellen Fällen der niedermolekularen Polysaccharide der pH-Werts des Pflanzenauszugs auf 2,5-3,5 eingestellt wird, wobei der gefällte zweite Niederschlag in dem Dispersen Extrakt System (DES) verbleibt;

Pyrrolizidinalkaloide-Reinigung mittels Entfernung von Pyrrolizidinalkaloiden (PAs) aus dem Dispersen Extrakt System des Primärextrakts, wobei für die Entfernung, insbesondere stufenweise Entfernung, von Pyrrolizidinalkaloiden (PAs) aus dem Dispersen Extrakt System, das Disperse Extrakt System mit einem Pyrrolizidinalkaloide-Adsorber versetzt wird und der Pyrrolizidinalkaloide-Adsorber die Pyrrolizidinalkaloide (PAs) aus dem Dispersen Extrakt System des Primärextrakts sorbiert und der zweite Niederschlag der niedermolekularen Polysaccharide im Dispersen Extrakt System des Primärextrakts vorliegt.

Ein Raffinat ist von einem Suspensions System bzw. dem Pflanzenauszug trennbar. Durch die differentielle Fällung der niedermolekularen Polysaccharide kann ein Disperses Extrakt System (DES), bestehend aus zweitem Niederschlag und Primärextrakt, entstehen.

Das abgetrennte Raffinat kann nebst unlöslichen Bestandteilen von Symphytum, die ursprünglich in einer Suspension kolloidal gelöst vorliegenden, hochmolekulare Polysaccharide, als ersten Niederschlag aufweisen. Das Raffinat kann zudem aufgesaugte Extraktrückstände aufweisen. Unter Polysacchariden sind optisch aktive Heteropolysaccharide, die aus Monosacchariden von Furanose- oder Pyranosestruktur mit Äther- oder Glykosidbindung aufgebaut sind und deren saure oder basische Funktionen ggf. mit einem Eiweiss und/oder Peptid gekoppelt sein können (Peptidoglykane oder Glykoproteine), zu verstehen. Der erste Niederschlag kann neben den hochmolekularen Polysacchariden noch andere inerte Begleitstoffe enthalten.

Die hochmolekularen Polysaccharide sind aus einem Suspensions System aus Symphytum durch eine erste differentielle Fällung als erster Niederschlag ausfällbar und mit dem Raffinat abtrennbar. Hierbei kann gleichzeitig eine erste Abreicherung der PAs stattfinden. Bei einer solchen ersten differentiellen Fällung bleiben niedermolekulare Polysaccharide im Pflanzenauszug bzw. im Suspensions System aus Symphytum kolloidal gelöst. Die niedermolekularen Polysaccharide können somit nach dem Trennvorgang vom Raffinat, im entstandenen Pflanzenauszug (Primärextrakt) für die nächste Bearbeitungsstufe zur Verfügung stehen, also von PAs gereinigt werden.

Vermutlich sind alle pflanzlichen Polysaccharide aus sich wiederholenden Untereinheiten aufgebaut, die dann als kleinere Bausteine ihre Wirksamkeit entfalten. Bei Polysacchariden mit niedrigem Molekulargewicht, also niedermolekulare Polysaccharide, ist aufgrund einer beschleunigten Resorption eine effizientere Wirkung zu erwarten als bei solchen mit höherem Molekulargewicht, also hochmolekulare Polysaccharide. Es wurde beschrieben, dass das Molekulargewicht über Veränderungen der Sekundär-und Tertiärstruktur auch das Wirkprofil zu beeinflussen vermag. Frühere Arbeiten lassen ebenso den Schluss zu, dass insbesondere die erwünschten niedermolekularen Polysaccharide die Wirkung anderer Inhaltsstoffe synergistisch oder additiv steigern, weshalb die erste differentielle Fällung hochmolekularer Polysaccharide, aus Sicht bisheriger Erkenntnisse, als eine Massnahme zur Entfernung unerwünschter inerter Begleitstoffe zu verstehen ist. Dadurch werden die unerwünschten, hochmolekularen Polysaccharide aus dem Suspensions System entfernt und die niedermolekularen Polysaccharide, die zu den gewünschten Extraktivstoffen gehören, bleiben im Pflanzenauszug (Primärextrakt) kolloidal gelöst, erhalten. Die niedermolekularen Polysaccharide können auch als bedeutsame, kolloidal gelöste Extraktivstoffe des Pflanzenauszugs (Primärextrakts) aufgefasst werden.

Das differentielle Fällen von niedermolekularen Polysacchariden als zweiten Niederschlag aus dem Pflanzenauszug (Primärextrakt) kann auch als zweite differentielle Fällung bezeichnet werden. Der zweite Niederschlag kann auch als Präzipitat bezeichnet werden.

Der gefällte zweite Niederschlag verbleibt im neu entstanden Dispersen Extrakt Systems (DES) des Primärextrakts.

In dem Primärextrakt kann durch die (zweite) differentielle Fällung eine Präzipitatphase entstehen. Bereits in der Präzipitatphase kann simultan mit dem Ausfällen der niedermolekularen Polysaccharide eine (zweite) Abreicherung an PAs stattfinden. Der zweite Niederschlag muss nicht vom Primärextrakt getrennt werden. Vielmehr können überraschenderweise PAs direkt aus dem Dispersen Extrakt System (Primärextrakt + zweiter Niederschlag), ohne vorgängige Trennung des Niederschlags, entfernt werden.

Der Begriff Pyrrolizidinalkaloide-gereinigt bzw. Pyrrolizidinalkaloide-Reinigung bedeutet, dass mindestens ein PA aus einer Phase, Extraktionsphase, einem Extrakt, Primärextrakt bzw. einer Lösung entfernt wird.

Die Pyrrolizidinalkaloide-Reinigung kann in einem sogenannten Eintopf-Verfahren durchgeführt werden. Das Eintopf-Verfahren ist ein vereinfachter bzw. optimierter Prozess, bei dem für die PA-Reinigung der Primärextrakt und der zweite Niederschlag (Päzipitat), als Disperses Extrakt System (DES), in einer Prozessanlage vorliegen. Die beiden Komponenten im Dispersen Extrakt System des Primärextrakts können in einzelnen Phasen der Herstellung eines Endprodukts auch voneinander getrennt werden. Der Begriff "Eintopf-Verfahren" bezieht sich hauptsächlich auf die PAs-Reinigung des entstandenen gesamten Dispersen Extrakt Systems (DES) des Primärextrakts. Dadurch kann das Verfahren der PAs-Reinigung mit wenigen Arbeitsschritten ermöglicht werden. Überraschenderweise hat sich gezeigt, dass nahezu vollständig die PAs selektiv entfernt werden können, wenn im DES des Primärextrakts beispielsweise im Kontaktverfahren und durch schonende Rührwirkung, der feindisperse zweite Niederschlag neben dem Primärextrakt verbleibt und in Schwebe gehalten wird. Diese Phase kann auch Reinigungsphase genannt werden. Das Disperse Extrakt System (DES) des Primärextrakts kann als disperse Mischung aus dem zweiten Niederschlag und einem Primärextrakt bezeichnet werden.

Unerwarteterweise muss der zweite Niederschlag für die PA-Reinigung nicht vom Primärextrakt B getrennt werden, wie beispielsweise in EP 3 159 002 beschrieben. EP 3 159 002 beschreibt ein alternatives (Teil-)Verfahren zur Pyrrolizidinalkaloid-Reinigung von Symphytum-Extrakten, bei dem der Niederschlag und die Extraktphase voneinander getrennt werden und separat voneinander gereinigt werden. Durch das Eintopf-Verfahren können daher nicht nur die Anzahl an Verfahrensschritten verringert, sondern zusätzlich kann auch die Ausbeute des Verfahrens erhöht werden, da keine Verluste bei der Trennung und/oder separaten PAs-Reinigung entstehen können. Damit kann das PAs-gereinigte DES des Primärextrakts, in Anwesenheit aller Wirkkomponeten, durch das hocheffiziente Eintopf-Verfahren der weiteren Verarbeitung des Endprodukts, in flüssiger oder fester Darreichungsform, als Spezialextrakt zugeführt werden.

Somit bedeutet dies eine prozesstechnische Verbesserung der Verfahrensstufe und eine Optimierung des gesamten Verfahrens zur PA-Reinigung und zudem lassen sich signifikante Zeit- und Kostenersparnisse realisieren. Während oder im Anschluss des Eintopf-Verfahrens kann, sofern dies erforderlich ist, in einzelnen Phasen oder zur Herstellung eines Endproduktes, der zweite Niederschlag des DES vom Primärextrakt getrennt werden.

Die Herstellung von Spezialextrakten gemäss dem Stand der Technik verlaufen hauptsächlich über mehrstufige Verfahren. Da die Zwischenprodukte von Symphytum Spezialextrakt bisher isoliert aufgereinigt werden mussten, sind solche Vorgehensweisen bei den Reinigungsschritten mit einem enormen Verbrauch von Lösungsmitteln und zudem mit technisch hohem Aufwand und langen Herstellungszeiten verbunden, wodurch hohe Produktionskosten entstehen. Eine neue Alternative stellt deshalb das erfindungsmässe mehrstufige Eintopf-Verfahren bei der Herstellung von Spezialextrakten aus Symphytum in einer entsprechenden Prozessanlage dar. Es beruht ohne jegliche Isolierungsschritte auf dem Reinigungsverfahren während einer zweiten differentiellen Fällung aus einem Pflanzenauszug (Primärextrakt) und im Anschluss daran auf dem weiteren Reinigungsverfahren an dem durch die Fällung entstehenden Dispersen Extrakt System (DES) des Primärextrakts.

Der zweite Niederschlag kann als feindisperser Niederschlag die niedermolekularen Polysaccharide aufweisen. Ähnlich wie der erste Niederschlag kann der zweite Niederschlag neben den Polysacchariden andere Begleitstoffe wie Proteine. enthalten. Durch das feindisperse Vorliegen des zweiten Niederschlags kann die Oberfläche des Niederschlags vergrössert werden, was wiederum die Pyrrolizidinalkaloid-Reinigung des Niederschlags erleichtern kann.

Für die Entfernung, insbesondere für die stufenweise Entfernung, insbesondere im Wesentlichen selektive Entfernung, von Pyrrolizidinalkaloiden (PAs) aus dem Suspensions System, aus dem Pflanzenauszug (Primärextrakt) und/oder aus dem Dispersen Extrakt System (DES) des Primärextrakts, insbesondere in einem Eintopf-Verfahren, kann das DES des Primärextrakts, bzw. das Suspensions System, bzw. der Pflanzenauszug mit einem Pyrrolizidinalkaloide-Adsorber versetzt werden. Der PAs-Adsorber kann die PAs direkt und umfassend aus dem DES des Primärextrakts durch eine sorptive Bindung abreichern.

Die Entfernung der PAs kann eine und stufenweise PA-Reinigung sein.

Der PA-Adsorber kann mindestens ein Adsorbens oder ein Adsorbensgemisch aufweisen. Der PAs-Adsorber kann mindestens ein PA oder ein Gemisch mehrerer PAs sorptiv binden. Dadurch kann eine Vielzahl an bzw. im Wesentlichen alle PAs aus dem DES des Primärextrakts entfernen werden.

Der PA-Adsorber kann insbesondere als Feststoff vorliegen und/oder an einem Feststoff gebunden sein.

Als Pyrrolizidinalkaloide-Adsorber können beispielsweise Ionenaustauscher; insbesondere Kationenaustauscher, insbesondere ein Kationenaustauscher in saurer Form; Tonmineralien, Cellulose, Kieselgel, Kieselsäure Aluminiumoxid, Kieselgur, Stärke; einzeln und/oder als Gemisch dienen. Der Kationenaustauscher kann auf der Basis eines organischen Polymerharzes ausgestaltet sein, wobei der Kationenaustauscher insbesondere stark saure Sulfonsäuregruppen (-SO3H) auf der Polymeroberfläche aufweisen kann. Die Sulfonsäuregruppen können auch Ankergruppe genannt werden und können die allgemeine chemische Formel R-S03-H aufweisen, wobei der Rest R als eine vernetzte Polystyrol- Matrix verstanden werden kann.

PAs die im zweiten Niederschlag mit den niedermolekularen Polysacchariden als unerwünschte Begleitsubstanz vorliegen, können durch ein entstehendes Konzentrationsgefälle zwischen Niederschlag und Primärextrakt des DES (Konzentrationsgradienten im Vergleich zum PAs-gereinigten Primärextrakt) aus dem Niederschlag, insbesondere dem feindispersen Niederschlag, entfernt werden. Dadurch kann im Eintopf-Verfahren eine Synergiewirkung erzielt werden, bei der PAs aus dem zweiten Niederschlag und dem Primärextrakt bzw. dem DES des Primärextrakts diffundieren oder herausgelöst werden und in Folge durch den PAs-Adsorber sorptiv gebunden werden können. Dadurch kann die Diffusion der PAs gemäss dem Konzentrationsgradienten weiter begünstigt werden.

Der Pyrrolizidinalkaloide-Adsorber, insbesondere der Kationenaustauscher, kann nach der Adsorption der PAs, insbesondere nach Erreichen des Sättigungsgrades jeweils von dem DES des Primärextrakts, abgetrennt werden. Der PA-Adsorber kann durch Sedimentation oder/und Siebung, zur Einleitung einer nächsten Reinigungsstufe, abgetrennt werden.

Nach dem Trennen kann das DES durch eine Kolloidmühle oder ein Dispergiersystem in eine feindisperse Form desagglomeriert werden. Dadurch werden Agglomerate der niedermolekularen Polysaccharide-Kolloide verhindert bzw. verringert. Dadurch kann die Effektivität des Eintopf-Verfahrens erhöht werden, da zum einen die Oberfläche des zweiten Niederschlags die Austauschkinetik begünstigt, andererseits können beim Desagglomerieren zusätzliche PAs in das DES des Primärextrakts freigesetzt werden, die anschliessend vom Pyrrolizidinalkaloide-Adsorber gebunden und im Eintopf-Verfahren entfernt werden können.

Mit anderen Worten: Vor jeder weiteren Reinigungsstufe kann zur Verhinderung von Agglomeratbildung, der zweite differentielle Niederschlag vor der Zuführung von neuem PAs-Adsorber, auch zur Verbesserung der Rheologie, durch eine Kolloidmühle oder ein entsprechendes Rührsystem dispergiert werden.

Das DES des Primärextrakts kann zur Einleitung einer ersten Reinigungsstufe und ebenfalls zur Verbesserung der Rheologie und Austauschvorgänge, insbesondere mit einem niederpolaren Lösungsmittel oder Lösungmittelgemisch in einer Menge von 25-50% verdünnt werden. Dadurch kann die Viskosität der Extraktlösung eingestellt werden und die Diffusion und/oder Adsorption der PAs in der Extraktlösung erleichtert werden.

Die Dauer der Adsorption, also der sorptiven Bindung, der PAs am Adsorber bzw. Adsorbens kann Reaktionszeit, Absorptionszeit und/oder Einwirkzeit genannt werden. Der PA-Adsorber kann also im Wesentlichen während der Einwirkzeit in Schwebe gehalten werden. Die Einwirkzeit vor dem Abtrennen des PA-Adsorbers, also zur (mehrmaligen) Austausch- Gleichgewichtseinstellung zwischen PA-Adsorber und dem Dispersen Extrakt System kann mindestens von mindesten 2 h, insbesondere mindestens 4 h, insbesondere mindestens 5 h, betragen. Im Wesentlich kann sich die Einwirkzeit nach dem Erreichen eines Konzentrationsgleichgewichts der PAs in dem Primärextrakt richten. Mit anderen Worten: Im Wesentlichen richtet sich die Einwirkzeit nach der Kinetik der Austauschvorgänge und kann jeweils experimentell ermittelt werden.

Das DES des Primärextrakts, auch Substanzgemisch genannt, kann nach dem Abtrennen des PAs-Adsorbers, insbesondere des Kationenaustauschers, insbesondere nach dem Dispergieren, erneut mit PA-Adsorber, insbesondere Kationenaustauscher, versetzt werden. Beim erneuten Versetzten des Primärextrakts mit Adsorber wird neuer Adsorber verwendet. Der neue Adsorber hat eine höherer Bindungskapazität für PAs als der abgetrennt Adsorber nach der Einwirkzeit.

Der neue Adsorber kann nach einer erneuten Einwirkzeit von mindesten 2 h, insbesondere mindestens 4 h, insbesondere mindestens 5 h, von dem DES des Primärextrakts, abgetrennt werden. Anschliessend kann das DES erneut mit neuem Adsorber versetzt werden. Ein solcher Adsorptionszyklus kann mehrmals, beispielsweise mindestens zweimal, insbesondere mindestens dreimal, insbesondere mindestens viermal, insbesondere fünfmal, durchgeführt werden. Die Einwirkzeit bzw. Dauer der Adsorptionszyklen kann variieren.

Das sorptive Binden der PAs kann also in einem mehrstufigen Verfahren durchgeführt werden. Der PA-Adsorber, insbesondere der Kationenaustauscher, kann nach einem jeweils erfolgten Reinigungsschritt alternierend durch Siebung getrennt und frischer Kationenaustauscher zugeführt werden.

Beim differentiellen Fällen der niedermolekularen Polysaccharide, insbesondere bei einer zweiten differentiellen Fällung von Polysacchariden, wird der pH-Wert des Pflanzenauszugs (Primärextrakts) auf 2,5-3,5 eingestellt. Der zweite Niederschlag mit den niedermolekularen Polysacchariden kann sich dabei als feindisperser, Niederschlag bilden. Der feine zweite Niederschlag kann eine hellbeige Farbe aufweisen. Aufgrund der feindispersen Struktur kann der Niederschlag effizient von unerwünschten PAs gereinigt werden.

Mit anderen Worten: Beim zweiten differentiellen Fällen der niedermolekularen Polysaccharide aus dem Pflanzenauszug (Primärextrakt) ergibt sich ein feindisperser zweiter Niederschlag und somit das Disperse Extrakt System (DES) des Primärextrakts, in welchem sich nach einer gewissen Standzeit ein Agglomerat ausbilden kann. Das allfällig entstandene Agglomerat kann vor jeder Reinigungsstufe mittels Dispergiergeräten einer feindispersen Form zugeführt werden. Aufgrund der feindispersen Struktur kann das DES des Primärextrakts die PAs, durch eine günstige Freisetzungskinetik, die sorptive Bindung der PAs an dem PAs-Adsorber und somit eine effiziente PAs-Reinigung ermöglichen. Eine Fällung von Polysacchariden, beispielsweise mit Ethanol, führt einerseits zu einem gummiartigen Agglomerat, welches andererseits in dieser Form keine PAs-Abreicherung ermöglicht.

Aufgrund der Absenkung des pH-Wertes auf pH 2,5-3,5 durch elektrostatische Wechselwirkungen und entgegengesetzten Ladungszustände der Polymerkolloide, als pH-abhängige Interaktion, kann der zweite Niederschlag mit niedermolekularen Polysacchariden entstehen. Der zweite, beispielsweise kolloiddisperse, Niederschlag, auch Präzipitat niedermolekularer Polysaccharide bezeichnet, kann neben dem Primärextrakt darin als begleitende Wirkkomponente verbleiben. Durch den Zusatz von PAs-Adsorber, insbesondere Kationenaustauscher, kann in einer Präzipitatphase eine erste Stufe der Abreicherung von PAs eingeleitet werden. Die PAs-Abreicherung bedeutet, dass ein Vielstoffgemisch von PAs an dem PA-Adsorber, insbesondere dem Kationenaustauscher, durch sorptive Bindung, abgereichert wird. Unter sorptive Bindung sind ionische Bindungen oder auch nichtionische Adsorptionsvorgänge zu verstehen.

Der pH-Wert des Pflanzenauszugs (Primärextrakts), des Suspensions Systems und/oder des Dispersen Extrakt Systems (DES) des Primärextrakts, kann mit Hilfe eines Katalysators bzw. Ionenaustausches, insbesondere eines Kationenaustauschers, und/oder mit Hilfe einer Säure, insbesondere einer Mineralsäure und/oder einer organischen Säure, eingestellt werden.

Es hat sich die Verwendung des Kationenaustauschers als vorteilhaft erwiesen. Einerseits kann dies zur Reduktion von unerwünschten Kationen führen und andererseits kann gleichzeitig der pH-Wert abgesenkt werden, was wiederum eine nicht unerhebliche erste PA-Abreicherung in allen vorgenannten Bearbeitungsstufen ermöglicht. Die weiteren Bearbeitungsstufen werden durch die Synergie-Effekte des Kationenaustauschers somit positiv beeinflusst.

Der Katalysator, auch Kationenaustauscher genannt, kann ein saurer Katalysator/Kationenaustauscher auf gelförmiger Polymerbasis, insbesondere ein stark saures, gelförmiges Harz auf Polymerbasis in Perlenform, mit Sulfonsäure als funktionelle Gruppe, sein. Auf diese Weise kann der Kationenaustauscher einfach, beispielsweise durch Sedimentation und/oder Absieben, aus einer Flüssigkeit entfernt werden. Der Kationenaustauscher kann auch als PA-Adsorber fungieren. Der Kationenaustauscher kann also eine sorptive Bindung von PAs bewirken. Als Kationenaustauscher kann ein Kationenaustauscher mit stark sauren Sulfonsäuregruppen verstanden werden.

Der saure Kationenaustauscher kann Sulfonsäuregruppen (-SO3H) auf der Polymeroberfläche aufweisen. Auf diese Weise kann neben einer sorptiven Bindung von PAs ein Austausch von Kationen gegen H+ stattfinden. Der Ionenaustausch kann auf der Oberfläche des Kationenaustauschers stattfinden, wobei Kationen gebunden werden und der pH-Wert gesenkt wird. Durch die Reduktion des pH-Wertes können zum Erreichen des erforderlichen isoelektrischen Punkts, durch jeweils gezielt erfolgte Dosierungen des Kationenaustauschers, die Polysaccharide sowohl im Suspensions System als auch im daraus gewonnenen Pflanzenauszug (Primärextrakt), selektiv als ersten oder zweiten differentiellen Niederschlag gefällt werden. Zusätzlich können auch PAs durch sorptive Bindung am Kationenaustauscher bzw. Pyrrolizidinalkaloide-Adsorber schrittweise angelagert werden. Dadurch kann es zu einer Teilreduktion von unerwünschten PAs im Suspensions System, im Extrakt, insbesondere im Pflanzenauszug (Primärextrakt) und/oder im DES des Primärextrakts, kommen.

Der PAs-Adsorber, insbesondere der Kationenaustauscher, kann in monodisperser oder heterodisperser Form vorliegen. Monodisperse Form heisst, dass die Kationenaustauscherteilen bzw. Kationenaustauscherpartikel im Wesentlichen gleich gross sind. Der Kationenaustauscher kann als kugelförmige Perlen in einheitlicher Grösse vorliegen. Die sorptive Bindung der PAs kann durch die Form und Beschaffenheit des Kationenaustauschers beeinflusst werden

Zum Einstellen des pH-Wertes kann eine Mineralsäure oder organische Säure verwendet werden. Auch hier kommt es durch die Absenkung des pH-Wertes zu einer selektiven Fällung der Polysaccharide wie bereits beschrieben, jedoch ohne Abreicherung unerwünschter PAs.

Das Verfahren zur Herstellung eines Spezialextrakts aus einem PA-gereinigten Dispersen Extrakt Systems (DES) aus Symphytum weist die folgenden Schritte auf:
- Bereitstellen des Dispersen Extrakt Systems herstellbar wie beschrieben, wobei das Disperse Extrakt System nebeneinander einen pyrrolizidinalkaloide-gereinigten Primärextrakt und den zweiten Niederschlag im DES aufweist;
- Lösen des zweiten Niederschlags im Dispersen Extrakt System zum Symphytum-Spezialextrakt.

Wie bereits beschreiben, kann der zweite Niederschlag vor dem Lösen als feindisperser Niederschlag neben dem (PAs-gereinigten) Primärextrakt, insbesondere im Dispersen Extrakt Systems des Primärextrakts vorliegen. Als weitere Herstellungsvariante besteht die Möglichkeit, den zweiten differentiellen Niederschlag als Präzipitat vom Primärextrakt abzutrennen, beide Wirkstoffgruppen einer separaten PAs-Reinigung zu unterziehen und in Folge wieder zu einem Wirkstoffgemisch zu verarbeiten. Diese Vorgehensweise wird im EP 3 159002 beschrieben.

Der PAs-gereinigte Spezialextrakt aus Symphytum, also der Lösung aus zweitem Niederschlag und Primärextrakt, kann aufkonzentriert werden. Der Symphytum Spezialextrakt kann als Primärextrakt des PA-gereinigten und gelösten Dispersen Extrakt Systems bezeichnet werden.

Der aufkonzentrierte Symphytum-Spezialextrakt liegt als Fluidextrakt vor und kann als stabilisierter Fluidextrakt vorliegen.

Der Fluidextrakt weist maximal 0.5 ppm Pyrrolizidinalkaloide (PAs), insbesondere maximal 0.25 ppm Pyrrolizidinalkaloide (PAs), insbesondere maximal 0.1 ppm Pyrrolizidinalkaloide (PAs), auf.

Der konzentierte Spezialextrakt weist eine hohe Wirkstoffkonzentration auf. Unerwünschte bzw. inerte Extraktivstoffe werden bei der Herstellung selektiv entfernt. Dadurch kann der konzentrierte Spezialextrakt besser verträglich werden und kann bei der Zubereitung zur kutanen Anwendung (Unguenta, Salben) die umfangreichen Vorgaben und Anforderungen einer modernen Arzneiform weitgehend erfüllen. Des Weiteren kann die Zusammensetzung und die Menge der Inhaltsstoffe gegebenenfalls standardisiert und/oder quantifiziert werden. Dies garantiert eine gleichbleibende Qualität des konzentrierten Spezialextrakts.

Das Aufkonzentrieren kann beispielsweise mittels Destillation, insbesondere Vakuumdestillation, des PAs-gereinigte Symphytum-Spezialextrakts , erfolgen.

Der konzentrierte Symphytum-Spezialextrakt kann als Fluidextrakt quantifiziert werden. Für das Fluidextrakt kann ein Droge-Extrakt-Verhältnis eingestellt werden. Das Droge-Extrakt-Verhältnis kann 1:3,5 bis 1:4,5 betragen. Der konzentrierte Spezialextrakt mit einem Droge-Extrakt-Verhältnis von 1:3,5-4,5 kann einen PAs-Gehalt von maximal 0,5 ppm, insbesondere maximal 0,1 ppm aufweisen.

Das konzentrierte und quantifizierte Fertigprodukt des Spezialextrakts kann also ein PAs-gereinigter und quantifizierter und mit Ethanol stabilisierte Extrakt aus Symphytum sein.

Beim Lösen des zweiten Niederschlags im Primärextrakt kann der pH-Wert auf 5.5-7.0, insbesondere 5.5-6.5, insbesondere 6.2-6.5, eingestellt werden.

Der Fluidextrakt, insbesondere der Spezialextrakt 1:3,5-4,5 kann durch schonende Trocknung, insbesondere durch verhindern von qualitätsmindernden Einflüssen während der Trocknung, insbesondere durch Lyophilisation zu einem Trockenextrakt verarbeitet werden, also in bzw. zu einem Trockenextrakt überführt werden.

Die Trocknung des Spezialextrakts ist nebst einer Vereinfachung der Transportlogistik ein Instrument der Konservierung, zudem die Voraussetzung zum Schutz vor unkontrollierten Instabilitäten und dient zugleich der Gewährleistung einer gleichbleibenden Qualität während einer Lagerung,
Durch die Trocknung, des (konzentrierten) Fluidextrakts kann der Spezialextrakt als Trockenextrakt länger haltbar gemacht bzw. einfacher transportiert bzw. einfacher dosiert werden. Eine schonende Trocknung kann beispielsweise mittels Gefriertrocknung erreicht werden. Durch die schonende Trocknung können die Wirk- und Extraktivstoffe des Spezialextrakts erhalten und vor oxidativen Einflüssen geschützt werden.

Die bereits im PA-gereinigten Primärextrakt und somit im Spezialextrakt enthaltenen, beispielsweise kolloidal gelösten, niedermolekularen Polysaccharide können ausgefällt und zur separaten Trocknung, beispielsweise durch Lyophilisieren, vorgängig getrennt werden. Das Ausfällen der niedermolekularen Polysaccharide im PA-gereinigten Primärextrakt bzw. Spezialextrakt oder im konzentrierten Spezialextrakt kann durch Säurezusatz bis zu einem pH-Wert von 2,5-3,5 erfolgen. Das Ausfällen kann auch mit hochprozentigem Alkohol, insbesondere Ethanol, erfolgen. Der durch die Fällung schleimbefreite Flüssigextrakt, kann zur besseren Trocknung noch weiter aufkonzentriert und anschliessend ebenfalls lyophilisiert werden. Die beiden separat getrockneten Phasen, also die getrocknete im Wesentlichen polysaccharidfreie Fluidphase des Symphytum Spezialextrakts und die getrocknete Niederschlagsphase, können nach dem Trocknungsvorgang, insbesondere nach dem Lyophilisieren, als Trockenextrakt zusammengeführt werden. Nebst einer Vereinfachung der Transportlogistik werden allfällige Instabilitäten im getrockneten Spezialextrakt weitgehend unterbunden. Das Lyophilisat kann zur Weiterverarbeitung des getrockneten Spezialextrakts, in einem Lösungsmittel oder Lösungsmittelgemisch gelöst werden. Der Trockenextrakt kann einen PA-Gehalt von maximal 4 ppm, insbesondere maximal 0.8 ppm, aufweisen.

Der konzentrierte, quantifizierte und/oder getrocknete Spezialextrakt kann wie beschrieben herstellbar sein. Der Spezialextrakt aus Symphytum; insbesondere der Spezialextrakt aus Symphytum officinale, insbesondere aus Symphytum officinale L.; kann in der Therapie angewendet werden, insbesondere in unterschiedlich konzentriertere Form in der Therapie angewendet werden. Der Spezialextrakt aus Symphytum durch verschiedenen Zubereitungsformen in der kutanen Therapie angewendet werden. Es ist auch möglich, dass der Spezialextrakt aus Symphytum im Bereich Pflege und Kosmetik angewendet werden kann. Der Spezialextrakt kann als Wirkkomponente für die perkutane Verabreichungsform anwendbar sein. Der Spezialextrakt kann insbesondere in der Therapie von Schmerzen, Entzündungen, Schwellungen, bei Kniegelenkarthrose, akuten Muskelschmerzen, Bewegungseinschränkungen, Prellungen, Zerrungen, Verstauchungen, Gelenkarthrose, Rückenschmerzen, Muskelbeschwerden und/oder Gelenkbeschwerden angewendet werden.

Das Verfahren zur Herstellung eines Pflanzenauszugs (Primärextrakts) aus Symphytum weist die folgenden Schritte auf:
- Bereitstellen von frischen, gefrorenen und/oder getrockneten Pflanzenteilen von Symphytum;
- Versetzen der Pflanzenteile mit einem ersten Lösungsmittel und/oder ersten Lösungsmittelgemisch zur Herstellung einer Extraktionsphase, insbesondere einer ersten Extraktionsphase;
- optionales Versetzen der Extraktionsphase, insbesondere der ersten Extraktionsphase, mit einem zweiten Lösungsmittel oder Lösungsmittelgemisch zur Herstellung einer zweiten Extraktionsphase; und
- Dispergieren (der vorzerkleinerten Pflanzenteile) und/oder suspendieren (pulverisierter, getrockneter Pflanzenteile) der zweiten Extraktionsphase, insbesondere der ersten und/oder zweiten Extraktionsphase, insbesondere mit (vorzerkleinerten) Pflanzenteilen, zu einem feindispersen Suspensions System aus Symphytum;
- differentielles Fällen von (kolloidal gelösten) hochmolekularen Polysacchariden als ersten Niederschlag aus dem Suspensions System von Symphytum;
- Abtrennen des ersten Niederschlags und eines Extraktionsrückstands als Raffinat vom Pflanzenauszug (Primärextrakt).

Der Pflanzenauszug (Primärextrakt) kann in einem oben beschriebenen Verfahren verwendet werden.

Die frischen, gefrorenen und/oder getrockneten Pflanzenteile können oberirdische und/oder unterirdische Pflanzenteile von Symphytum aufweisen. Die getrockneten Pflanzenteile können auch in pulverisierter Form vorliegen.

Die Pflanzenteile von Symphytum können vorzerkleinert sein. Die Schnittgrösse der Symphytumpflanzenteile kann dabei 5-15 mm, insbesondere mindestens 5-10 mm, betragen.

Beim Versetzen der vorzerkleinerten Pflanzenteile kann das erste Lösungsmittel ein reines Lösungsmittel und/oder ein Lösungsmittelgemisch sein. Das Versetzen mit dem ersten Lösungsmittel kann einer ersten Extraktion, also einer ersten Extraktionsphase, entsprechen.

Als Lösungsmittel, insbesondere ein erstes und/oder zweites Lösungsmittel kann ein Gemisch aus Wasser und niedermolekularem Alkohol, wie beispielsweise Ethanol, Methanol, Propanol, Isopropanol usw., verwendet werden, welcher maximal zehn C-Atome aufweist.

Beim Zerkleineren der Pflanzenteile kann als Vorbereitungsschritt zum Suspensions System eine Nasszerkleinerung angewandt werden. Mit anderen Worten: Bei der Herstellung des Suspensions Systems können die vorzerkleinerten Pflanzenteile durch eine Nasszerkleinerung zu einer feindispersen Form zerkleinert werden.

Der erste Niederschlag kann neben den hochmolekularen Polysacchariden auch andere inerte und unerwünschte Begleitsubstanzen, wie bspw. Proteine, aufweisen. Dieses können (gesamthaft) als Raffinat abgetrennt werden.

Nach einem Erreichen eines Extraktionsgleichgewichts, bspw. in einer dritten Extraktionsphase, kann die Trennung des Pflanzenauszugs (Primärextrakt) vom Raffinat durch Abpressen und/oder Absaugen und/oder Anwendung anderer Trennprozesse vorgenommen werden. Unter Raffinat sind nebst aufgesaugtem Pflanzenauszug, unlösliche Pflanzenteile (Drogenrückstand) sowie im vorliegenden Falle zusätzlicher erster Niederschlag unerwünschter hochmolekularer Polysaccharide und andere inerte Bestandteile zu verstehen.

Durch eine Fest-Flüssig-Extraktion der Pflanzenteile von Symphytum sowie dem nachfolgend differentiellen Fällen erfolgt das zusammenhängendes Abtrennen einer ersten Schleimfällung unerwünschter hochmolekularer Polysaccharide, einhergehend mit einer Abtrennung der gesamten übrigen Festphase (Raffinat) aus einem Suspensions System und die Gewinnung des daraus resultierenden Pflanzenauszugs (Primärextrakt).

Bei der Nasszerkleinerung des Extraktionsgutes (Symphytum Pflanzenteile) kann die Partikelgrösse von 5-15 mm auf 0.1-0.5 mm reduziert werden. Die Bildung des Suspensions Systems kann, durch eine Nasszerkleinerung der Pflanzenteile von Symphytum bei einer Temperatur von 20°C - 35°C erfolgen. Die Pflanzenzellen werden bei der Nasszerkleinerung durch die Schneid- und Scherkräfte vermehrt aufgeschlossen, es ergibt sich ein Suspensions System, welches ein rasches Extraktionsgleichgewicht durch zeitverkürzte Diffusionsvorgänge ermöglicht.

Die Extraktionsphase, insbesondere die erste Extraktionsphase, kann durch Digerieren und/oder Perkolieren der (vorzerkleinerten) Planzenteile hergestellt werden. Die Extraktionsphase kann insbesondere bei einer Temperatur von 45°C-80°C, insbesondere 50°C-70°C, insbesondere 53°C-57°C und zudem bei einer Feststoffkonzentration von 9-11% stattfinden.

Unter Perkolation versteht man die Gewinnung von Auszügen aus kleingeschnittenen Pflanzenteilen mittels eines kontinuierlich fliessenden oder durchsickernden Lösungsmittel. Perkolieren kann als Sickerextraktions verstanden werden. Die Perkolation kann vorzugsweise mit vorzerkleinerten Pflanzenteile von Symphytum durchgeführt werden.

In der Extraktionsphase, insbesondere in der ersten Extraktionsphase, insbesondere bei vorgenannten erhöhten Temperaturen können bei gleichzeitiger Anwesenheit von Alkohol, Keime stark reduziert werden. Ebenso ist damit durch den Ethanolgehalt von 70% V/V, die Deaktivierung pflanzeneigener und zersetzender Enzyme, und zudem durch das einhergehende Denaturieren von Proteinen für die Lagerstabilität des nachfolgend entstehenden Fluidextraktes von Bedeutung.

Die Extraktionsphase, insbesondere die erste Extraktionsphase, kann optional vor der Nasszerkleinerung, also vor dem Dispergieren, mit einem zweiten Lösungsmittel versetzt werden. Das zweite Lösungsmittel kann eine höhere Polarität aufweisen als das erste Lösungsmittel. Das Lösungsmittel kann ein Lösungsmittelgemisch sein. Durch die Zugabe des zweiten Lösungsmittels zur ersten Extraktionsphase kann eine zweite Extraktionsphase gebildet werden. Mit anderen Worten: Vor der Zugabe des zweiten Lösungsmittels liegt die ersten Extraktionsphase vor, wobei nach der Zugabe des zweiten Lösungsmittel (und durch die Nasszerkleinerung zu einem Suspensions System) die zweite Extraktionsphase entsteht.

Das erste Lösungsmittel kann ein niederpolares Lösungsmittel, insbesondere Ethanol 65 - 75% V/V, vorzugsweise 70%V/V sein. In der zweiten Extraktionsphase kann durch Zugabe von einem höherpolaren zweiten Lösungsmittel, insbesondere Ethanol 7,5 -12,5% V/V, vorzugsweise 10%V/V, mit einem Auszugsmittel, also Lösungsmittel, mit erhöhter Polarität, korrespondierend zu einem erniedrigten Ethanolgehalt von 45 - 55%V/V, insbesondere 50%V/V, extrahiert werden. Durch die Polaritätserhöhung des Lösungsmittels der zweiten Extraktionsphase können die Polysaccharide quellen, kolloidal gelöst und bei einer Feststoffkonzentration von 5 - 7,5% unter höherviskosen Bedingungen, mittels Rührmazeration, extrahiert werden. Die Nasszerkleinerung kann mittels einer Kolloidmühle, oder einer anderen Dispergiermaschine ermöglicht werden. Es ist ebenso auch möglich die ersten Extraktionsphase durch eine Nasszerkleinerung feindispers zu suspendieren.

Für die (erste) differentielle Fällung des ersten Niederschlags aus dem Suspensions System, also kolloidal gelöster, insbesondere hochmolekularer, Polysaccharide kann der pH-Wert des Suspensions System auf 4 - 5, insbesondere auf 4.5, eingestellt werden. Die erste differentielle Fällung kann vor dem Abtrennen des Raffinats stattfinden. Vor dem Abtrennen des Raffinats vom Pflanzenauszug (Primärextrakt) kann eine dritte Extraktionsphase gebildet werden. In dieser dritten Extraktionsphase, die bei einem pH-Wert korrespondierend dem isoelektrischen Punkt (IEP) der hochmolekulare Polysaccharide stattfindet, verbessern sich die Extraktionsbedingungen. Diese Verbesserung kann auf einer Reduktion der Viskosität der Extraktionsphase, insbesondere der dritten Extraktionsphase im Vergleich zur zweiten Extraktionsphase, und/oder auf einer Veränderung der Polarität und/oder der Selektivität der Extraktionsphase beruhen. In der dritten Extraktionsphase können weitere Extraktivstoffe aus Symphytum gelöst und extrahiert werden.

Der pH-Wert der dritten Extraktionsphase kann mit Hilfe eines Kationenaustauschers, einer Mineralsäure und/oder einer organischen Säure eingestellt werden, wie oben beschrieben. Der Kationenaustauscher kann durch Austauscheffekte das Absenken des pH-Wertes und darüber hinaus durch sorptive Bindung eine erste Abreicherung unerwünschter PAs bewirken.

Der Pflanzenauszug (Primärextrakt) kann über eine sogenannte Stufenextraktion hergestellt werden, bei der die Extraktivstoffe in der ersten Extraktionsphase, anschliessenden in der zweiten Extraktionsphase und letztendlich in der dritten Extraktionsphase gelöst bzw. extrahiert werden.

In diesem Text bedeutet der Begriff Pyrrolizidinalkaloide mindestens ein Pyrrolizidinalkaloid und kann das gesamte Gemisch von mehr als 100 verschiedene Pyrrolizidinalkaloiden umfassen. Der Pflanzenauszug (Primärextrakt) kann als Extraktstufe mit pH 4-5, und das Disperse Extrakt System (DES) des Primärextrakts kann als Extraktstufe mit pH 2-3 bezeichnet werden.

Das Disperse Extrakt System kann als disperses Extraktsystem und das Suspensions System als Suspensionssystem, oder auch als suspendiertes (Extrakt-)System, bezeichnet werden.

Weitere bevorzugte Ausführungsformen gehen aus den abhängigen Patentansprüchen hervor. Die Merkmale der Verfahrensansprüche sind sinngemäss miteinander und den Vorrichtungsansprüchen kombinierbar und umgekehrt.

### Beispiel

Unterirdische Teile von Symphytum, in einer Schnittgrösse von 5 - 10 mm werden in einer ersten niederviskosen Extraktionsphase mit Ethanol 70%V/V, bei einer Feststoffkonzentration von 9 -11%, durch digerieren oder perkolieren extrahiert.

In der zweiten, höherviskosen Extraktionsphase wird bei einem Ethanolgehalt von 50% V/V, bei einer Feststoffkonzentration von 5 - 7,5%, durch Nasszerkleinerung auf eine Partikelgrösse von 0,1 - 0,5 mm, ein Suspensions System hergestellt.

In der dritten, niederviskosen Extraktionsphase erfolgt unter Säurezusatz oder mit Hilfe eines Kationenaustauschers, im Suspensions System eine erste differentielle Ausfällung und ggf. Adsorption unerwünschter Extraktivstoffe (Pyrrolizidinalkaloide - kurz PAs) bei einem pH-Wert von 4 - 5. Im Anschluss daran erfolgt durch geeignete Methoden, die Trennung der unlöslichen Bestandteilen (Extraktionsrückstand/Ausfällung) als Raffinatphase und dem Pflanzenauszug als Primärextrakt.

In einem nachfolgenden "Eintopf-Verfahren", zur Einleitung einer zweiten differentiellen Ausfällung erwünschter Extraktivstoffe (Präzipitat/zweiter Niederschlag) unter Einstellung des pH-Wertes 2,5 - 3,5 des Pflanzenauszugs (Primärextrakts) durch zufügen eines Kationenaustauschers, erfolgt in Anwesenheit und Verbleib der zweiten, feindispersen differentiellen Ausfällung (Präzipitat/zweiter Niederschlag), durch gleichzeitige sorptive Bindung unerwünschter Extraktivstoffe (PAs), unter Rühren, der erste Verfahrensschritt vom Dispersen Extrakt System (DES) des Primärextrakts.

Zur Durchführung der weiteren Reinigungsschritte wird das vorgenannte "Eintopf-Verfahren" in demselben Reaktionsbehälter einer Prozessanlage, unter Beibehaltung des entstandenen Dispersen Extrakt Systems des Primärextrakts in dem zur weiteren Entfernung von PAs, der jeweils angereicherte Kationenaustauscher stufenweise entfernt und alternierend durch neuen Kationenaustauscher ersetzt wird.

Das im "Eintopf-Verfahren" gereinigte DES vom Primärextrakt enthält Rest-PAs von maximal 0,1 ppm und wird durch Einstellen auf einen pH-Wert von 5,5 - 6,5 sowie durchs Lösen des zweiten dispersen Niederschlags zum Symphytum Spezialextrakt, nachfolgend mittels Vakuumdestillation konzentriert, mit Ethanol 96% V/V stabilisiert und anschliessend durch Lyophilisierung zum getrockneten SMC-Beinwell Spezialextrakt mit einem Droge-Extrakt-Verhältnis (DEV) von 2,4 - 1,9 : 1, verarbeitet.

## Patentansprüche

1. **Verfahren** zur Pyrrolizidinalkaloide-Reinigung von einem Pflanzenauszug aus Symphytum, aufweisend die folgenden Schritte:
- Bereitstellen von einem Pflanzenauszug aus Symphytum;
wobei ein Raffinat von dem Pflanzenauszug abgetrennt wird, und wobei das Raffinat hochmolekulare Polysaccharide in einem ersten Niederschlag aufweist, und die hochmolekularen Polysaccharide aus einem Suspensions System aus Symphytum in einer ersten differentiellen Fällung ausgefällt werden, indem der pH-Wert des Suspensions Systems auf 4 - 5 eingestellt wird,
- differentielles Fällen von niedermolekularen Polysacchariden als zweiten Niederschlag aus einem nach dem Trennvorgang vom Raffinat entstandenen Pflanzenauszug (Primärextrakt), und beim differentiellen Fällen der niedermolekularen Polysaccharide der pH-Werts des Pflanzenauszugs auf 2,5-3,5 eingestellt wird;
wobei der gefällte zweite Niederschlag in einem Dispersen Extrakt System des Primärextrakts **verbleibt;**
- Pyrrolizidinalkaloide-Reinigung mittels Entfernung von Pyrrolizidinalkaloiden (PAs) aus dem Dispersen Extrakt System des Primärextrakts, wobei für die Entfernung, insbesondere stufenweise Entfernung, von Pyrrolizidinalkaloiden (PAs) aus dem Dispersen Extrakt System, das Disperse Extrakt System mit einem Pyrrolizidinalkaloide-Adsorber versetzt wird und der Pyrrolizidinalkaloide-Adsorber die Pyrrolizidinalkaloide (PAs) aus dem Dispersen Extrakt System des Primärextrakts sorbiert und der zweite Niederschlag der niedermolekularen Polysaccharide im Dispersen Extrakt System des Primärextrakts vorliegt.

2. Verfahren gemäss Anspruch 1, wobei der zweite Niederschlag als feindisperser Niederschlag die niedermolekularen Polysaccharide aufweist.

3. Verfahren gemäss einem der vorherigen Ansprüche, wobei für die Entfernung das Disperse Extrakt System mit einem Kationenaustauschers in saurer Form, versetzt wird; und
wobei der Kationenaustauscher, die Pyrrolizidinalkaloide (PAs) aus dem Dispersen Extrakt System des Primärextrakts sorbiert und der zweite Niederschlag derniedermolekularen Polysaccharide im Dispersen Extrakt System des Primärextrakts vorliegt.

4. Verfahren gemäss Anspruch 3, wobei der Pyrrolizidinalkaloide-Adsorber, insbesondere der Kationenaustauscher, nach der Sorption der Pyrrolizidinalkaloide (PAs) vom Dispersen Extrakt System des Primärextrakts abgetrennt wird und nach dem Abtrennen das Disperse Extrakt System in eine feindisperse Form dispergiert wird.

5. Verfahren gemäss emem der Ansprüche 3-4, wobei das Disperse Extrakt System des Primärextrakts nach dem Abtrennen des Pyrrolizidinalkaloide-Adsorbers, insbesondere des Kationenaustauschers, insbesondere nach dem Dispergieren, erneut mit Pyrrolizidinalkaloide-Adsorber, insbesondere Kationenaustauscher, versetzt wird.

6. Verfahren gemäss Anspruch 1, wobei der pH-Wert des Pflanzenauszugs mit Hilfe eines Kationenaustauschers in saurer Form, einer Mineralsäure und/oder einer organischen Säure eingestellt wird, insbesondere wobei der Kationenaustauscher auf der Basis eines organischen Polymerharzes ausgestaltet ist und wobei der Kationenaustauscher insbesondere Sulfonsäuregruppen auf der Polymeroberfläche aufweist.

7. **Verfahren** zur Herstellung eines **Spezialextrakts** aus einem Pyrrolizidinalkaloide-gereinigten Dispersen Extrakt System des Primärextrakts, aufweisend dieSchritte:
- Bereitstellen des Dispersen Extrakt System des Primärextrakts herstellbar gemäss einem der Ansprüche 1-6,
wobei das Disperse Extrakt System des Pyrrolizidinalkaloide-gereinigten Primärextrakt und den zweiten Niederschlag **nebeneinander** aufweist;
- Lösen des zweiten Niederschlags in dem Pyrrolizidinalkaloidgereinigten, Dispersen Extrakt System des Primärextrakts zum Symphytum Spezialextrakt.

8. Verfahren gemäss Anspruch 7, wobei das Verfahren den folgenden Schritt aufweist:
- Aufkonzentrieren des Spezialextrakts;
wobei der aufkonzentrierte Spezialextrakt als Fluidextrakt vorliegt, insbesondere wobei der Fluidextrakt quantifiziert wird, insbesondere wobei für den Fluidextrakt ein Droge-Extrakt-Verhältnis (DEV) eingestellt wird, insbesondere wobei der Fluidextrakt ein Droge-Extrakt-Verhältnis (DEV) von 1:3,5 bis 1:4,5 aufweist;
wobei der Fluidextrakt maximal 0.5 ppm Pyrrolizidinalkaloide (PAs), insbesondere maximal 0.25 ppm Pyrrolizidinalkaloide (PAs), insbesondere maximal 0.1 ppm Pyrrolizidinalkaloide (PAs), aufweist; und/oder wobei beim Lösen des zweiten Niederschlags im Dispersen Extrakt System der pH-Wert auf 5.5-7.0, insbesondere 5.5-6.5, eingestellt wird.

9. Verfahren gemäss einem der Ansprüche 7-8, wobei der Fluidextrakt zu einem Trockenextrakt verarbeitet wird, insbesondere wobei der Fluidextrakt mittels Lyophilisation zum Trockenextrakt verarbeitet wird.

10. Verfahren gemäss Anspruch 9, wobei die im Fluidextrakt enthaltenen niedermolekulare Polysaccharide ausgefällt werden und von der Fluidphase getrennt werden,
wobei die ausgefällten niedermolekulare Polysaccharide separat von der abgetrennten Fluidphase getrocknet, insbesondere lyophilisiert, werden; und insbesondere wobei die abgetrennte Fluidphase vor dem Trocknen zusätzliche aufkonzentriert wird; und
wobei die beiden separat getrockneten Phasen nach dem Trocknungsprozess, insbesondere nach dem Lyophilisieren, als Trockenextrakt zusammengeführt werden.

11. **Verfahren nach einem der Ansprüche 1-6 zur** Herstellung eines **Pflanzenauszugs** aus Symphytum, aufweisend die folgenden Schritte:
- Bereitstellen von frischen, gefrorenen und/oder getrockneten Pflanzenteilen von Symphytum;
- Versetzen der Pflanzenteile mit einem ersten Lösungsmittel zur Herstellung einer Extraktionsphase, insbesondere einer ersten Extraktionsphase;
- Versetzen der Extraktionsphase, insbesondere der ersten Extraktionsphase, mit einem **zweiten Lösungsmittel;**
- dispergieren und/oder suspendieren der Extraktionsphase zu einem feindispersen Suspensions System aus Symphytum;
- differentielles Fällen von hochmolekularen Polysacchariden als ersten Niederschlag aus dem Suspensions System von Symphytum;
- Abtrennen des ersten Niederschlags und eines Extraktionsrückstands als Raffinat von dem Pflanzenauszug.

12. Verfahren gemäss Anspruch 11, wobei die Extraktionsphase, insbesondere die ersten Extraktionsphase, durch Digerieren und/oder Perkolieren der Planzenteile, insbesondere der vorzerkleinerten Planzenteile, hergestellt wird, insbesondere wobei die Extraktionsphase bei einer Temperatur oberhalb Raumtemperatur, insbesondere bei einer Temperatur von 45°C bis 80°C, insbesondere 50°C - 70°C, insbesondere 54-56°C, hergestellt wird.

13. Verfahren gemäss einem der Ansprüche 11-12, wobei die Extraktionsphase, insbesondere die erste Extraktionsphase, vor dem Dispergieren zu einem feindispersen Suspensions System mit einem zweiten Lösungsmittel versetzt wird, insbesondere wobei das zweite Lösungsmittel eine höhere Polarität aufweist als das erste Lösungsmittel, insbesondere wobei durch die Zugabe des zweiten Lösungsmittels zur ersten Extraktionsphase eine zweite Extraktionsphase gebildet wird.

14. Verfahren gemäss einem der Ansprüche 11-12, wobei für die differentielle Fällung des ersten Niederschlags aus dem Suspensions System aus Symphytum, insbesondere vor dem Abtrennen des Raffinats, der pH-Wert des Suspensions System auf 4 - 5, insbesondere auf 4.5, eingestellt wird, insbesondere wobei vor dem Abtrennen des Raffinats vom Pflanzenauszug eine dritte Extraktionsphase gebildet wird.

## Claims

1. A method for pyrrolidine alkaloid purification of a plant extract from symphytum, comprising the following steps:
- Providing a plant extract from symphytum;
wherein a raffinate is separated from the plant extract, and wherein the raffinate has high-molecular polysaccharides in a first precipitate, and the high-molecular polysaccharides are precipitated from a suspension system of symphytum in a first differential precipitation by adjusting the pH-value of the suspension system to 4 - 5,
- differential precipitation of low-molecular polysaccharides as a second precipitate from a plant extract (primary extract) formed after the separation process from the raffinate, and in the differential precipitation of the low-molecular polysaccharides the pH-value of the plant extract is adjusted to 2,5 -3,5;
wherein the precipitated second precipitate remains in a disperse extract system of the primary extract;
- pyrrolizidine alkaloid purification by means of the removal of pyrrolizidine alkaloids (PAs) from the disperse extract system of the primary extract, wherein for the removal, in particular stepwise removal, of pyrrolizidine alkaloids (PAs) from the disperse extract system, the disperse extract system is mixed with a pyrrolizidine alkaloids adsorber and the pyrrolizidine alkaloids adsorber sorbs the pyrrolizidine alkaloids (PAs) from the disperse extract system of the primary extract and the second precipitate of the low-molecular polysaccharides is present in the disperse extract system of the primary extract.

2. The method according to claim 1, wherein the second precipitate has the low-molecular polysaccharides as a finely dispersed precipitate.

3. The method according to anyone of the preceding claims, wherein the disperse extract system is mixed with a cation exchanger in acidic form for the removal; and
wherein the cation exchanger sorbs the pyrrolizidine alkaloids (PAs) from the disperse extract system of the primary extract and the second precipitate of the low-molecular polysaccharides is present in the disperse extract system of the primary extract.

4. The method according to claim 3, wherein the pyrrolizidine alkaloids adsorber, in particular the cation exchanger, is separated from the disperse extract system of the primary extract after the sorption of the pyrrolizidine alkaloids (PAs) and, after separation, the disperse extract system is dispersed into a finely dispersed form.

5. The method according to anyone of the claims 3 - 4, wherein the disperse extract system of the primary extract is mixed again with pyrrolizidine alkaloids adsorber, in particular cation exchanger, after separation of the pyrrolizidine alkaloids adsorber, in particular cation exchanger, in particular after the dispersion.

6. The method according to claim 1, wherein the pH-value of the plant extract is adjusted by means of a cation exchanger in acidic form, a mineral acid and/or an organic acid, in particular wherein the cation exchanger is structured on the basis of an organic polymer resin and wherein the cation exchanger has, in particular, sulfonic acid groups on the polymer surface.

7. A method for producing a special extract from a pyrrolidine alkaloid-purified disperse extract system of the primary extract, comprising the steps:
- Providing the disperse extract system of the primary extract producible according to anyone of the claims 1 - 6,
wherein the disperse extract system has the pyrrolizidine alkaloid-purified primary extract and the second precipitate side by side;
- Dissolving the second precipitate in the pyrrolizidine alkaloid-purified disperse extract system of the primary extract to the symphytum special extract.

8. The method according to claim 7, the method comprising the following step:
- Concentrating the special extract;
wherein the concentrated special extract is present as a fluid extract, in particular wherein the fluid extract is quantified, in particular wherein a drug-extract-ratio (DEV) is adjusted for the fluid extract, in particular wherein the fluid extract has a drug-extract-ratio (DEV) of 1:3,5 to 1:4,5;
wherein the fluid extract has a maximum of 0.5 ppm pyrrolizidine alkaloids (PAs), in particular a maximum of 0.25 ppm pyrrolizidine alkaloids (PAs), in particular a maximum of 0.1 ppm pyrrolizidine alkaloids (PAs); and/or wherein when dissolving the second precipitate in the disperse extract system, the pH-value is adjusted to 5.5 - 7.0, in particular 5.5 - 6.5.

9. The method according to anyone of the claims 7 -8, wherein the fluid extract is processed to a dry extract, in particular wherein the fluid extract is processed to the dry extract by means of lyophilization.

10. The method according to claim 9, wherein the low-molecular polysaccharides contained in the fluid extract are precipitated and separated from the fluid phase,
wherein the precipitated low-molecular polysaccharides are dried, in particular lyophilized, separately from the separated fluid phase; and
in particular wherein the separated fluid phase is additionally concentrated before drying; and
wherein the two separately dried phases are brought together as a dry extract after the drying process, in particular after lyophilization.

11. The method according to anyone of the claims 1 - 6 for producing a plant extract from symphytum, comprising the following steps:
- Providing fresh, frozen and/or dried plant parts of symphytum;
- Mixing the plant parts with a first solvent for producing an extraction phase, in particular a first extraction phase;
- Mixing the extraction phase, in particular the first extraction phase, with a second solvent;
- dispersing and/or suspending the extraction phase to a finely dispersed suspension system of symphytum;
- differential precipitation of high-molecular polysaccharides as the first precipitate from the suspension system of symphytum;
- Separation of the first precipitate and an extraction residue as raffinate from the plant extract.

12. The method according to claim 11, wherein the extraction phase, in particular the first extraction phase, is produced by digesting and/or percolating the plant parts, in particular the pre-crushed plant parts, in particular wherein the extraction phase is produced at a temperature above room temperature, in particular at a temperature of 45°C to 80°C, in particular 50°C - 70°C, in particular 54 - 56°C.

13. The method according to anyone of the claims 11 - 12, wherein the extraction phase, in particular the first extraction phase, before dispersing to a finely dispersed suspension system, is mixed with a second solvent, in particular wherein the second solvent has a higher polarity than the first solvent, in particular wherein a second extraction phase is formed by the addition of the second solvent to the first extraction phase.

14. The method according to anyone of the claims 11 - 12, wherein for the differential precipitation of the first precipitate from the suspension system of symphytum, in particular before separating the raffinate, the pH-value of the suspension system is adjusted to 4 - 5, in particular to 4.5, in particular wherein a third extraction phase is formed before separating the raffinate from the plant extract.

## Revendications

1. Un procédé pour la purification d'alcaloïdes de pyrrolizidine d'un extrait végétal de symphytum, comprenant les étapes suivantes :
- Mise à disposition d'un extrait végétal de symphytum ;
dans lequel un raffinat est séparé de l'extrait végétal, et dans lequel le raffinat présente des polysaccharides de poids moléculaire élevé dans un premier précipité, et les polysaccharides de poids moléculaire élevé sont précipités à partir d'un système de suspension de symphytum dans une première précipitation différentielle en ajustant le pH du système de suspension à 4 - 5,
- Précipitation différentielle de polysaccharides de faible poids moléculaire en tant que deuxième précipité à partir d'un extrait végétal (extrait primaire) formé après le processus de séparation du raffinat, et lors de la précipitation différentielle des polysaccharides de faible poids moléculaire, le pH de l'extrait végétal est ajusté à 2,5 - 3,5 ;
dans lequel le deuxième précipité reste dans un système d'extrait dispersé de l'extrait primaire ;
- Purification d'alcaloïdes de pyrrolizidine au moyen de l'élimination d'alcaloïdes de pyrrolizidine (PAs) du système d'extrait dispersé de l'extrait primaire, dans laquelle, pour l'élimination, en particulier l'élimination par étapes, d'alcaloïdes de pyrrolizidine (PAs) du système d'extrait dispersé, le système d'extrait dispersé est mélangé avec un adsorbeur d'alcaloïdes de pyrrolizidine et l'adsorbeur d'alcaloïdes de pyrrolizidine sorbe les alcaloïdes de pyrrolizidine (PAs) du système d'extrait dispersé de l'extrait primaire et le deuxième précipité des polysaccharides de faible poids moléculaire est présent dans le système d'extrait dispersé de l'extrait primaire.

2. Le procédé selon la revendication 1, dans lequel le deuxième précipité présente les polysaccharides de faible poids moléculaire sous forme de précipité finement dispersé.

3. Le procédé selon l'une des revendications précédentes, dans lequel le système d'extrait dispersé est mélangé avec un échangeur de cations sous forme acide pour l'élimination ; et
dans lequel l'échangeur de cations sorbe les alcaloïdes de pyrrolizidine (PAs) du système d'extrait dispersé de l'extrait primaire et le deuxième précipité de polysaccharides de faible poids moléculaire est présent dans le système d'extrait dispersé de l'extrait primaire.

4. Le procédé selon la revendication 3, dans lequel l'adsorbeur d'alcaloïdes de pyrrolizidine, en particulier l'échangeur de cations, est séparé du système d'extrait dispersé de l'extrait primaire après la sorption des alcaloïdes de pyrrolizidine (PAs) et, après la séparation, le système d'extrait dispersé est dispersé sous une forme finement dispersée.

5. Le procédé selon l'une des revendications 3 à 4, dans lequel le système d'extrait dispersé de l'extrait primaire est mélangé à nouveau avec l'adsorbeur d'alcaloïdes de pyrrolizidine, en particulier l'échangeur de cations, après la séparation de l'adsorbeur d'alcaloïdes de pyrrolizidine, en particulier l'échangeur de cations, en particulier après la dispersion.

6. Le procédé selon la revendication 1, dans lequel le pH de l'extrait végétal est ajusté à l'aide d'un échangeur de cations sous forme acide, d'un acide minéral et/ou d'un acide organique, en particulier dans lequel l'échangeur de cations est conçu à base d'une résine polymère organique et dans lequel l'échangeur de cations présente en particulier des groupes acides sulfonique sur la surface du polymère.

7. Un procédé de production d'un extrait spécial à partir d'un système d'extrait dispersé purifié d'alcaloïdes de pyrrolizidine de l'extrait primaire, comprenant les étapes de :
- Mise à disposition le système d'extrait dispersé de l'extrait primaire pouvant être produit selon l'une des revendications 1 à 6,
dans lequel le système d'extrait dispersé présente côte à côte l'extrait primaire purifié d'alcaloïdes de pyrrolizidine et le deuxième précipité ;
- Dissolution du deuxième précipité dans le système d'extrait dispersé purifié d'alcaloïdes de pyrrolizidine de l'extrait primaire à l'extrait spécial de symphytum.

8. Le procédé selon la revendication 7, dans lequel le procédé présente l'étape suivante :
- Concentrer l'extrait spécial ;
dans lequel l'extrait spécial concentré se présente sous la forme d'un extrait fluide, en particulier dans lequel l'extrait fluide est quantifié, en particulier dans lequel un rapport drogue-extrait (DEV) est ajusté pour l'extrait fluide, en particulier dans lequel l'extrait fluide présente un rapport drogue-extrait (DEV) de 1:3,5 à 1:4,5 ;
dans lequel l'extrait fluide présente au maximum 0.5 ppm d'alcaloïdes de pyrrolizidine (PAs), en particulier au maximum 0.25 ppm d'alcaloïdes de pyrrolizidine (PAs), en particulier au maximum 0.1 ppm d'alcaloïdes de pyrrolizidine (PAs) ; et/ou dans lequel, lors de la dissolution du deuxième précipité dans le système d'extrait dispersé, le pH est ajusté à 5.5 - 7.0, en particulier 5.5 - 6.5.

9. Le procédé selon l'une des revendications 7 à 8, dans lequel l'extrait fluide est transformé en un extrait sec, en particulier dans lequel l'extrait fluide est transformé en un extrait sec par lyophilisation.

10. Le procédé selon la revendication 9, dans lequel les polysaccharides de faible poids moléculaire contenus dans l'extrait fluide sont précipités et séparés de la phase fluide,
dans lequel les polysaccharides de faible poids moléculaire précipités sont séchés, en particulier lyophilisés, séparément de la phase fluide séparée ; et en particulier dans lequel la phase fluide séparée est concentrée en plus avant le séchage ; et
dans lequel les deux phases séchées séparément sont réunies sous forme d'extrait sec après le processus de séchage, en particulier après la lyophilisation.

11. Le procédé selon l'une des revendications 1 à 6 pour la production d'un extrait végétal à partir de symphytum, présentant les étapes suivantes :
- Mise à disposition de parties de plantes fraîches, congelées et/ou séchées de symphytum ;
- Mélanger les parties de plantes avec un premier solvant pour produire une phase d'extraction, en particulier une première phase d'extraction ;
- Mélanger la phase d'extraction, en particulier la première phase d'extraction, avec un deuxième solvant ;
- Disperser et/ou mettre en suspension la phase d'extraction en un système de suspension finement dispersé de symphytum ;
- Précipitation différentielle de polysaccharides de poids moléculaire élevé comme premier précipité du système de suspension de Symphytum ;
- Séparation du premier précipité et d'un résidu d'extraction sous forme de raffinat de l'extrait végétal.

12. Le procédé selon la revendication 11, dans lequel la phase d'extraction, en particulier la première phase d'extraction, est produite par digestion et/ou percolation des parties de plantes, en particulier les parties de plantes pré-broyées, en particulier dans lequel la phase d'extraction est produite à une température supérieure à la température ambiante, en particulier à une température de 45°C à 80°C, en particulier de 50°C à 70°C, en particulier de 54 à 56°C.

13. Le procédé selon l'une des revendications 11 à 12, dans lequel la phase d'extraction, en particulier la première phase d'extraction, avant de se disperser en un système de suspension finement dispersé, est mélangée avec un deuxième solvant, en particulier dans lequel le deuxième solvant présente une polarité plus élevée que le premier solvant, en particulier dans lequel une deuxième phase d'extraction est formée par l'addition du deuxième solvant à la première phase d'extraction.

14. Le procédé selon l'une des revendications 11 à 12, dans lequel pour la précipitation différentielle du premier précipité du système de suspension de symphytum, en particulier avant la séparation du raffinat, le pH du système de suspension est ajusté à 4 - 5, en particulier à 4.5, en particulier dans lequel une troisième phase d'extraction est formée avant la séparation du raffinat de l'extrait végétal.
